# EUROPEAN PATENT SPECIFICATION

(11) **EP 2 586 463 B1**
(45) Date of publication and mention of the grant of the patent: **17.08.2016**
(21) Application number: 11792458.9
(22) Date of filing: 07.06.2011
(51) Int. Cl.: A61K 9/10, A61K 9/51, A61K 31/4745, A61K 31/573, A61K 47/48, A61P 35/00

(54) **PHARMACEUTICAL MULTIMERIC PARTICLES, AND MANUFACTURING METHOD FOR SAME**
PHARMAZEUTISCHE MULTIMERE PARTIKEL UND HERSTELLUNGSVERFAHREN DAFÜR
PARTICULES MULTIMÈRES PHARMACEUTIQUES ET LEUR PROCÉDÉ DE FABRICATION

(30) Priority: 11.06.2010 JP 2010133486
(43) Date of publication of application: 01.05.2013
(73) Proprietor: Japan Science and Technology Agency, Kawaguchi-shi, Saitama 332-0012 (JP)
(72) Inventor: KASAI, Hitoshi, Sendai-shi Miyagi 982-0003 (JP); NAKANISHI, Hachiro, Sendai-shi Miyagi 980-0813 (JP); BABA, Koichi, Mino-shi Osaka 562-0031 (JP); OIKAWA, Hidetoshi, Sendai-shi Miyagi 982-0003 (JP); MURAKAMI, Tastuya, Kyoto-shi Kyoto 616-8142 (JP); IMAHORI, Hiroshi, Kyoto-shi Kyoto 604-8093 (JP); HASHIDA, Misturu, Kyoto-shi Kyoto 606-0845 (JP); OH, Isamu, Sennan-gun Osaka 598-0092 (JP)
(74) Representative: Jones, Nicholas Andrew
(86) International application number: PCT/JP2011/063084
(87) International publication number: WO 2011/155501

(56) References cited:
- WO-A1-2009/013466
- WO-A1-2009/075391
- WO-A1-2010/053101
- WO-A2-2007/075825
- CN-A- 101 628 919
- JP-A- 6 079 168
- HITOSHI KASAI ET AL: "Creation of Pure Nanodrugs and Their Anticancer Properties", ANGEWANDTE CHEMIE INTERNATIONAL EDITION, vol. 51, no. 41, 8 October 2012 (2012-10-08), pages 10315-10318, XP055100363, ISSN: 1433-7851, DOI: 10.1002/anie.201204596
- YOUQING SHEN ET AL.: 'Prodrugs Forming High Drug Loading Multifunctional Nanocapsules for Intracellular Cancer Drug Delivery' JOURNAL OF AMERICAN CHEMICAL SOCIETY, [Online] vol. 132, no. 12, 10 March 2010, pages 4259 - 4265, XP055069801 Retrieved from the Internet: <URL:http://pubs. acs.org/doi/abs/10.1021/ja909475m>
- KEREN HABA ET AL.: 'Single-Triggered Trimeric Prodrugs' ANGEWANDTE CHEMIE INTERNATIONAL EDITION, [Online] vol. 44, 2005, pages 716 - 720, XP055069808 Retrieved from the Internet: <URL:http://onlinelibrary. wiley.com/doi/10.1002/anie.200461657/abstra ct>
- MASAO SUZUKI ET AL.: 'Preparation of Poly (amic acid) Nanoparticles Using the Reprecipitation Method and Their Imidization' JAPANESE JOURNAL OF POLYMER SCIENCE AND TECHNOLOGY vol. 59, no. 10, 2002, pages 637 - 641, XP055100531
- HITOSHI KASAI ET AL.: 'Jisedai Yakuzai 'Pure Nano Drug' no Sosei to sorera no Yakuri Koka' 2010 NENDO DAI 10 KAI TAGEN BUSSHITSU KAGAKU KENKYUSHO KENKYU HAPPYOKAI KOEN YOKOSHU 13 January 2011, page 63

## Description

### Technical Field

The present invention relates to pharmaceutical multimeric particles and a manufacturing method for the same.

### Background of the Invention

Organic particles are used in a variety of fields based on the properties such as high chemical activity, specific electronic state, good dispersion stability and the like.

For example, in the field of pharmaceuticals, poorly-soluble drugs are often used as aqueous suspension agent wherein pharmaceutical particles are dispersed in water. Since aqueous suspension agents exert the medicinal effect by gradually eluting the drug component from particles in the body, it is required that the particle size of the pharmaceutical particles is as small as possible in order to exert the medicinal effect efficiently.

In addition, organic pigments wherein poorly-soluble organic dyes are microparticulated are used for various purposes such as toner for electrophotography, ink-jet ink, color filter, paint, printing ink, and the like. Regarding these organic pigments, it is also desired from the viewpoint of improving in various qualities such as color reproducibility, image quality, dispersion stability, and the like that the particles are smaller and have uniform particle size.

Organic particles exert a superior performance in various uses including these by making them smaller, therefore development of technology is proceeding with nano-microparticulation of organic compounds.

Traditionally, particles of poorly-soluble drugs and pigments are produced by mechanically pulverizing coarse particles with ball mill, etc. However, it is difficult to decrease the particle size to nanometer scale, also the particle size varies greatly.

Meanwhile, when a drug is administered to human body, a poorly-soluble drug is often used as an aqueous suspension agent wherein the drug is dispersed in water.

Having a property that the fine particulate drug component dispersed in water remains in the body, aqueous suspension agents are effective in exerting medicinal effects locally or over a long time, and used as oral preparation, dermatological topical preparation, nasal topical preparation, ophthalmologic topical preparation, and the like according to the purpose.

Aqueous suspension agents are usually produced by dispersing microparticulated drug into water with using a surfactant and the like. However the particle size of the drug obtained by pulverization is relatively large and the particle size varies greatly, thus there was a problem that large particle components settle out during long-term storage or by a rapid change of environment such as temperature. In addition, due to the variation of particle size, the quality as aqueous suspension agents is subject to variation, therefore there was a problem that even in the drug produced by the same manufacturing method, the retention time in some cases is too short to exert its effect, while in other cases it is so long that there is a threat of producing side effects. Thus methods for producing pharmaceutical particles wherein the particle size is small and the particle size distribution is narrow are desired.

On the other hand, Camptothecin that is an anticancer agent and specific derivatives thereof have been known (US2007041093, US20100233190, WO2008/034124, US6011042, WO98/035940). Camptothecin and most of its derivatives are extremely insoluble in water, so clinical application of this drug is constrained. As water-soluble Camptothecin derivatives, 9-dimethylaminomethyl-10-hydroxycamptothecin (Topotecan), 7-[(4-methylpiperazino)methyl]-10,11-ethylenedioxycamptothe cin, 7-[(4-methylpiperazino)methyl]-10,11-methylenedioxycamptoth ecin, and 7-ethyl-10-[4-(1-piperidino)-1-piperidino]carbonyloxycampto thecin (CPT-11) are exemplified. The use of CPT-11 (Irinotecan/Camptosar, Resistered trade mark) for human was approved by the U.S. Food and Drug Administration in June 1996.

In 1984, US4473692 by Miyasaka et al. disclosed that the active substance of CPT-11 is 10-hydroxy-7-ethylcamptothecin (SN-38). The water-solubility of SN-38 is extremely poor, so it was not directly administered to human cancer patients. In recent years, it is reported that in human patients, SN-38 is further metabolized to form inactivated glucuronide species. NK012 that is a polymer micelle preparation of SN-38 is being developed, but micellar nanoparticles have various problems, so its practical use is questioned.

In addition, clinical development of liposome preparation (LE-SN38) of SN-38 is carried out, however it cannot pass the primary tumor response end point yet. Therefore desired is the method for development of new drug having SN-38 as a medicinally effective component and using novel DDS.

On the other hand, the present inventors have developed a novel method for reprecipitation technique to produce a dispersion solution of nanometer scale particles (JP-A 6-079168). This is to produce a dispersion solution of ultrafine particles of organic material by pouring a solution of said organic material dissolved in good solvent into poor solvent. According to this method, it is possible to produce a dispersion solution of fine particles of the compounds 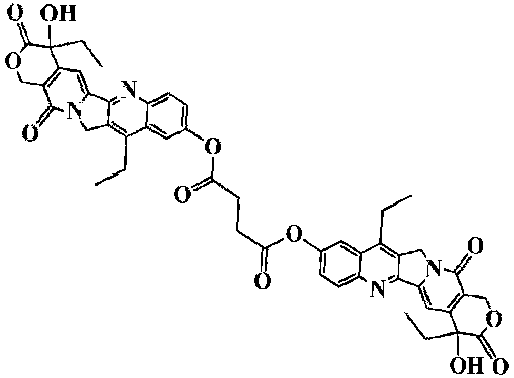 and

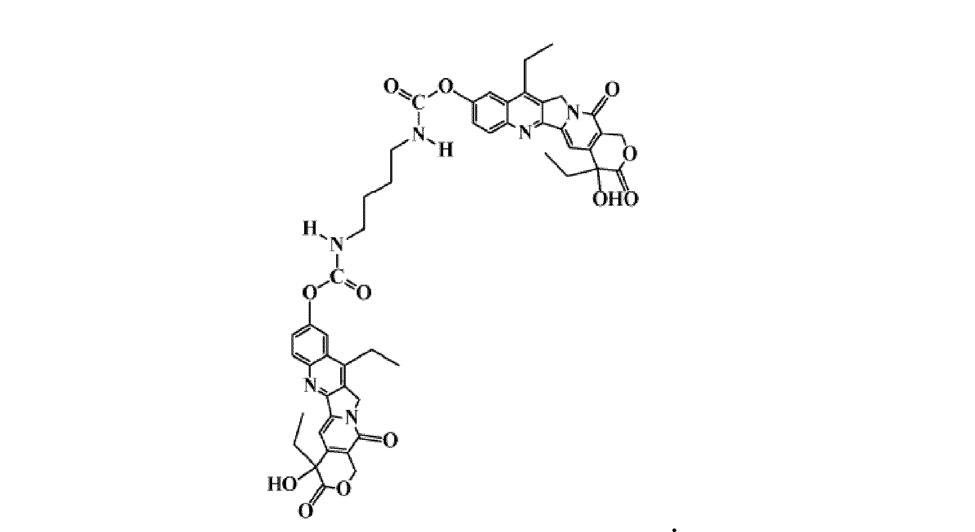

### Prior Art

### Patent Document

patent document 1: US2007041093
patent document 2: US20100233190
patent document 3: WO2008/034124
patent document 4: US6011042
patent document 5: WO98/035940
patent document 6: US4473692
patent document 7: JP6-079168A

Youqing Shen et al., J. Am. Chem. Soc., 2010, 132, 4259-4265 discloses prodrugs forming high drug loading multifunctional nanocapsules for intracellular cancer drug delivery.

CN101628919 discloses camptothecin and a self-emulsifying medicine precursor of a derivative thereof.

Keren Haba et al. , Angew. Chem. Int. Ed. , 2005, 44, 716-720 discloses single-triggered trimeric prodrugs.

WO2009/075391 discloses nanoparticles containing pioglitazone or a salt thereof and a biocompatible polymer, a pharmaceutical preparation containing the nanoparticles for the prophylaxis or treatment of arteriosclerotic diseases, and a stent carrying the nanoparticles.

Masao Suzuki et al., Japanese J. Pol. Sci. Tech., 2002, 59, 637-641 discloses the preparation of poly(amic acid) nanoparticles using the reprecipitation method and their imidization.

WO2007/075825 discloses lipophilic di(anticancer drug) compounds, compositions that include the compounds, and methods for treating a cell proliferative disease using the compounds.

WO2009/013466 discloses a process and device for the precipitation of organic compounds.

WO2010/053101 discloses an eye drop which can achieve high intraocular migration of a medicinal agent contained therein, wherein the eye drop is characterised by comprising particles composed of a hydrophobic or liposoluble prodrug that can become hydrophilic or water-soluble upon hydrolysis.

Hitoshi Kasai et al., 2010 NENDO DAI 10 KAI TAGEN BUSSHITSU KAGAKU KENKYUSHO KENKYU HAPPYOKAI KOEN YOKOSHU, January 2011, page 63 and Hitoshi Kasai et al., Angew. Chem. Int. Ed. , 2012, 51, 10315-10318 disclose the preparation of pure nanodrugs, including SN-38, and their physiological effects.

### Disclosure of Invention

### Problems to be resolved by the Invention

According to this method, it is possible to manufacture a dispersion solution of fine crystals of the compounds listed in appended claim 1. However, in some kind of drugs, this method cannot give particles having satisfactory particle size and particle size distribution, therefore a method applicable to the compounds listed in appended claim 1 is desired. Depending on a combination of organic compound and poor solvent, there was a case where it is difficult to obtain particles having sufficiently small particle size.

Therefore, the object of the present invention is to provide organic particles containing pharmaceutical particles having small particle size and narrow particle size distribution and a manufacturing method for the same.

### Means of Solving the Problems

The present inventors have intensively investigated, and as the result, have found that the above-mentioned problems can be solved by using the pharmaceutical dimers identified above wherein two drugs are linked via biodegradable substituents, and finally completed the present invention.

That is, the first aspect of the present invention provides solid, nanoparticles of pharmaceutical dimer (pharmaceutical dimeric particles), wherein the pharmaceutical dimer is selected from the group consisting of 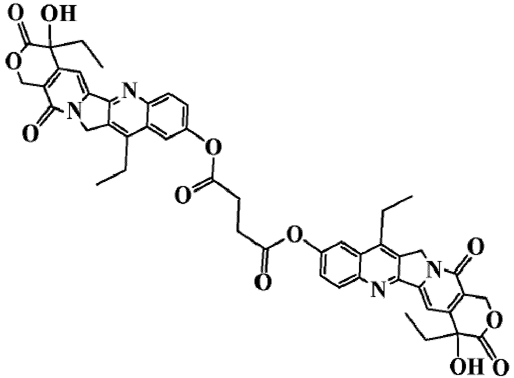 and 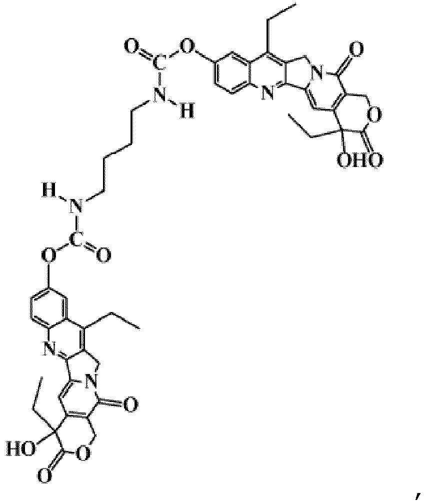 and said pharmaceutical dimeric particles are characterized by being obtained by pouring a solution of the pharmaceutical dimer dissolved in a water-miscible organic solvent into water.

The second aspect of the present invention provides a manufacturing method of the solid pharmaceutical dimeric nanoparticles identified above to solve the above-mentioned problem, wherein the manufacturing method of the pharmaceutical dimeric particles is characterized by pouring a solution of pharmaceutical dimer dissolved in a water-miscible organic solvent into water.

In a third aspect of the present invention, there is provided an anti-cancer agent comprising the solid pharmaceutical nanoparticle of the invention as the active ingredient.

### Effect of the Invention

According to the present invention, the pharmaceutical dimeric particles having small particle size and narrow particle size distribution can be obtained easily by using reprecitipation method established as a technique for producing superfine particles. The reprecitipation method can be applied to the compounds 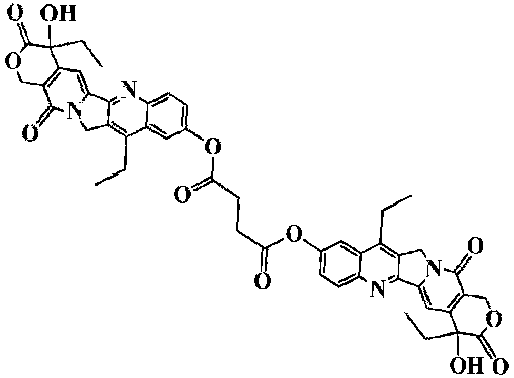 and 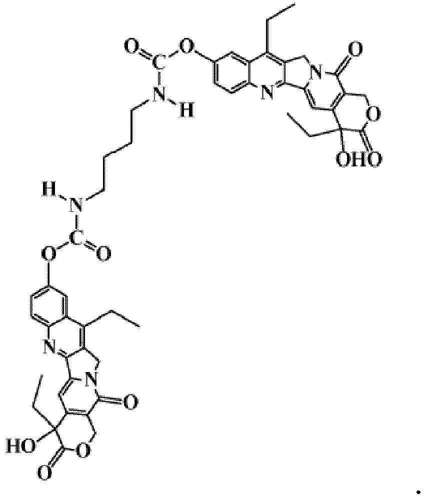 The pharmaceutical dimer regenerates the original drug by hydrolysis in vivo, that is, practically works as pharmaceutical particle itself, thus the medicinal effect and safety can be ensured. According to the present invention, organic particles having sufficiently small particle size and narrow particle size distribution can be provided. Since the particles of the present invention can be produced by using the technique of the reprecitipation method as it is, the particles can be obtained conveniently and at low cost. In addition, according to the manufacturing method of the present invention, particles having sufficiently small particle size can be obtained in the combination of an organic compound in which the particle size becomes big with a traditional reprecitipation method and a poor solvent.

### Brief Description of the Drawings

[Fig. 1] Figure 1 shows the particle size of dimer of SN-38 and succinic acid.
[Fig. 2] Figure 2 shows the particle size of dimer of SN-38 and tetramethylene diisocyanate.
[Fig. 3] Figure 3 shows the anticancer activity of nanoparticles of SN-38 dimer against HepG2.
[Fig. 4] Figure 4 shows the change in anticancer activity against HepG2 in aging of aqueous dispersion solution of SN-38 dimer nanoparticles
[Fig. 5] Figure 5 shows the effect of linking mode used for producing SN-38 dimer on anticancer activity against HepG2.

### Mode for Carrying Out the Invention

The present invention relates to preparing high quality pharmaceutical dimeric particles by dimerization of drug and application of reprecitipation method. Hereinafter, the present invention will be described in detail, and the basic manufacturing method of organic particles by reprecitipation method is disclosed in the aforementioned US2007041093, thus it can be referenced.

The particles manufactured in the present invention are dimers of a drug represented by 7-Ethyl-10-hydroxycamptothecin (SN-38).

In the present invention, the pharmaceutical dimer is one in which the drugs are linked to each other via a polyfunctional compound, wherein the polyfunctional compound is selected from succinic acid and tetramethylene diisocyanate.

Hereinafter, the pharmaceutical dimer is exemplified.

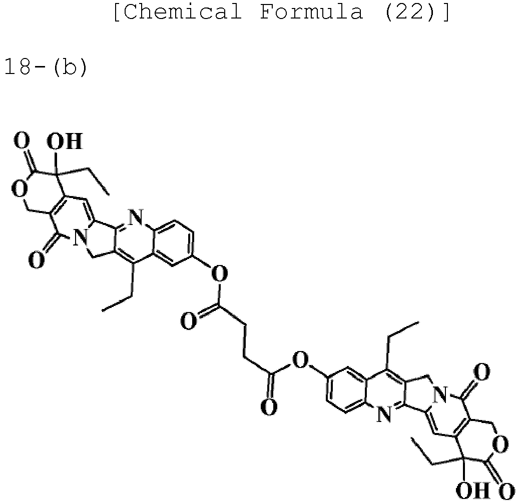

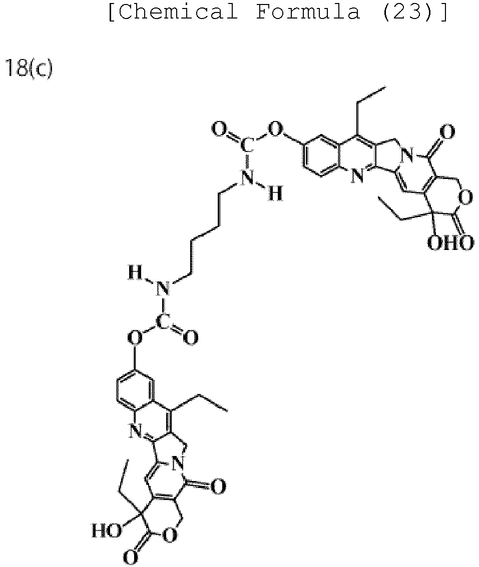

The pharmaceutical dimer of the present invention can be produced with a known method by a person skilled in the art. For example, it can be produced by reacting the drug with a polyfunctional compound selected from succinic acid and tetramethylene diisocyanate.

The pharmaceutical dimer can be produced, for example, based on the method described in US2007041093.

In producing the pharmaceutical dimeric particles of the present invention, first, pharmaceutical dimer is dissolved in an organic solvent which is a good solvent. As long as an organic solvent can dissolve the pharmaceutical dimer and is miscible with water, it can be appropriately selected as the organic solvent. As specific organic solvents, exemplified are acetone, tetrahydrofuran, dioxane, acetonitrile, methanol, ethanol, propanol, N-methylpyrrolidone, dimethyl sulfoxide, and these may be used alone or in a mixture. As organic solvent, acetone, ethanol and dimethyl sulfoxide are preferred from a viewpoint of solubility and safety.

Because concentration of pharmaceutical dimer dissolved in an organic solvent is a major factor affecting the particle size of particles to be formed, the concentration is appropriately changed depending on the desired particle size. Solution concentration is usually adjusted to around 0.1 to 15% by mass, and when the molecular weight is high, a solution of about 0.5% by mass is preferably prepared.

When a solution of pharmaceutical dimer in an organic solvent (hereinafter, simply referred to as organic solvent solution) is poured into water which is a poor solvent, pharmaceutical dimeric particles are formed in a condition dispersed in water. In the present invention, the solubility of pharmaceutical dimer in water becomes lower than that of original drug. As a result, the pharmaceutical dimer crystallizes more rapidly in water than the original drug, providing particles having smaller particle size and particle size distribution.

The amount of water wherein an organic solvent solution is poured into is usually more than 10 times based on the volume of pharmaceutical dimer. Although there is no upper limit, when water is too much, the concentration of the pharmaceutical dimeric particles becomes low and condensation is needed, thus usually the amount of water is not more than 100 times.

Since when the temperature of water wherein an organic solvent solution is poured into is changed, the particle size of the particles formed is also changed, the particle size of the pharmaceutical dimeric particles can be controlled by keeping the water at a given temperature. For example, when low temperature water is used, the particle size of the pharmaceutical dimeric particles tends to become large. Depending on the desired particle size, temperature is controlled usually within a range of -20°C to 60°C.

Pouring of organic solvent solution is preferred to carry out quickly in a short time into water under stirring condition in order to prevent variation of particle size. By continuing stirring for a while after pouring, an aqueous dispersion can be obtained wherein the crystallized or microparticulated pharmaceutical dimers are dispersed uniformly in water. The obtained pharmaceutical dimeric particles can be used directly with the state dispersed in water as an aqueous suspension agent, and further can be isolated as powder of fine particles by carrying out a solid-liquid separation operation such as filtration.

When used as an aqueous suspension agent, although the aqueous dispersion can be used directly depending on the intended use and the kind of good solvent used, the organic solvent added as good solvent is generally removed so as to improve the safety as a drug. The method for removing the organic solvent is not limited, and it can be removed from the aqueous dispersion by a known method. The organic solvent can be removed easily by distilling away under reduced pressure (or ordinary pressure), and can also be removed by using dialysis.

By conducting the above-mentioned operation, pharmaceutical dimeric particles having very small particle size and narrow particle size distribution can be obtained, that is, it is possible to produce pharmaceutical dimeric particles having narrow particle size distribution and large particle size by controlling the water temperature, kind of good solvent, the amount added, etc., and usually pharmaceutical dmeric particles having mean particle size of about 50nm to 10000nm can be produced by altering the production condition.

Pharmaceutical dimers are hydrolyzed in vivo to the form of original drug by the change in pH during transit through the living body and action of enzyme such as esterase. Therefore, the medicinal effect and safety of the pharmaceutical dimeric particles of the present invention can be ensured since they serve in vivo as the drug itself before dimerizing.

### Example 1

### Synthesis of SN-38 dimer (Compound 18-(b)) (succinic acid bis(7-ethylcamptothecin-10-yl) ester)

### <Esterification>

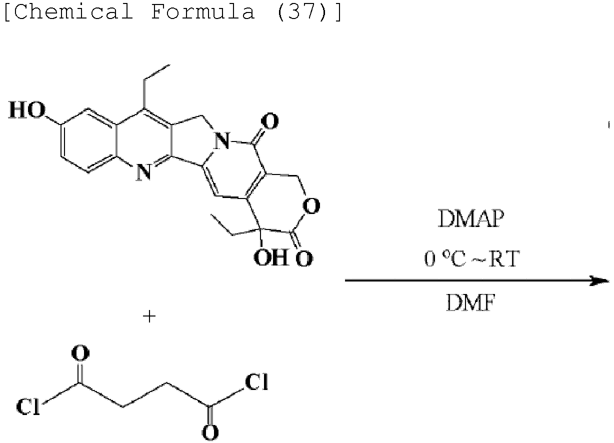

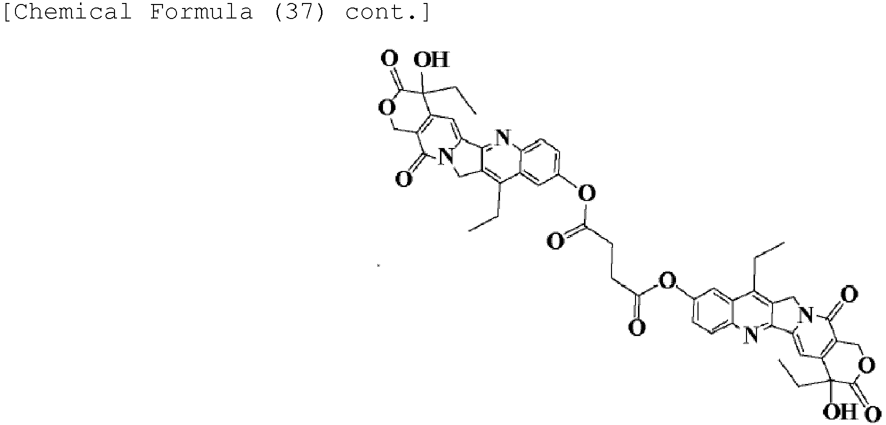

SN-38 (Compound 18-(a))(10 mg, 0.025 mmol) and 4-dimethylaminopyridine (DMAP: 7 mg, 0.058 mmol) were placed in two-necked eggplant flask, and after nitrogen substitution, 1 ml of dehydrated N,N-dimethylformamide (DMF) was added, followed by stirring for 30 minutes. After this eggplant flask is cooled in ice to 0°C, succinyl dichloride (2.6 *µ*l, 0.023 mmol) was added, and stirred for 4 hours with gradually warming to room temperature.

The stirred solution was transferred to separatory funnel, ethyl acetate was added, and washed more than 2 times with aqueous ammonium chloride solution. The solution was separated into aqueous phase and organic phase, each placed in Erlenmeyer flask, and the aqueous phase was confirmed with thin-layer chromatography (TLC) and the organic phase was filtered through absorbent cotton after dried over magnesium sulfate. The solvent of the solution was completely distilled away under reduced pressure, and the resulting crystals were washed with chloroform/methanol = 1/5 on a drain grating under suction filtration. The color of the purified crystals is white-purple, and yield is 55% for ester dimer of SN-38 (Compound 18-(b)).
¹HNMR (400MHz, DMSO,δ) : 0.87 (t, J = 7.4Hz, 6H), 1.26 (t, J = 7.2 Hz, 6H), 1.86 (s, 4H), 3.15 (s, 8H), 5.34(s, 3H), 5.43(s, 4H), 6.53 (s, 2H), 7.35(s, 2H), 7.73(s, 2H)
MS (ESI) : m/z = 867 (M+H⁺)

### Example 2

### Synthesis of SN-38 dimer (Compound 18-(c)) (butylene bis(7-ethylcamptothecin-10-yl)amide)

### <urethane bond>

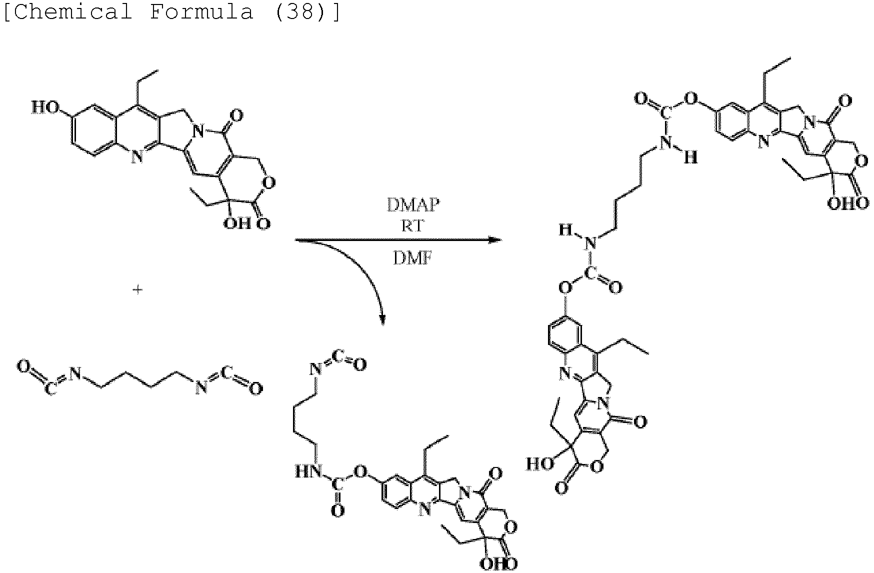

SN-38 20 mg (0.051 mmol) and DMAP (0.116 mmol) were placed in two-necked eggplant flask, and after nitrogen substitution, 1 ml of dehydrated DMF and tetramethylene diisocyanate 3 *µ*l (0.023 mmol) were added, followed by stirring at room temperature overnight. The stirred solution was transferred to separatory funnel, ethyl acetate was added, and washed several times with aqueous ammonium chloride solution. The solution was separated into aqueous phase and organic phase, each placed in Erlenmeyer flask, and the aqueous phase was confirmed with thin-layer chromatography (TLC) and the organic phase was filtered through absorbent cotton after dried over magnesium sulfate. Minimum amount of silica which was adsorbed to the filtrate was added, and the solvent was completely distilled away under reduced pressure, then purified by silica gel column chromatography with chloroform/methanol = 20/1 as developing solvent. The color of the purified crystals was yellow, and the yield of dimer of SN-38 and tetramethylene diisocyanate (Compound 18-(c)) was 42%.
¹HNMR (dimer : 400MHz,DMSO,δ) : 0.87 (t, J = 6.8Hz, 6H), 1.26 (t, J = 7.3Hz, 6H), 1.59(s, 4H), 1.86(q, J = 7.3Hz, 4H),3.14(q, J = 8.3Hz, 4H), 3.32(t, J = 21.9Hz,, 4H), 5.29 (s, 4H), 5.42(s, 4H), 6.51 (s, 2H), 7.30 (s, 2H), 7.66(q, J = 11 .7Hz, 2H), 7.92 (s, 2H), 8.01 (s, 2H), 8. 15 (d, J = 9.8Hz, 2H)
MS (ESI : dimer) : m/z = 924.8 (M+H⁺)

### Example 3 (comparative)

### Synthesis of SN-38 dimer (Compound 18-(d)) (bis(7-ethylcamptothecin-10-oxy)2-buten ether)

### <ether>

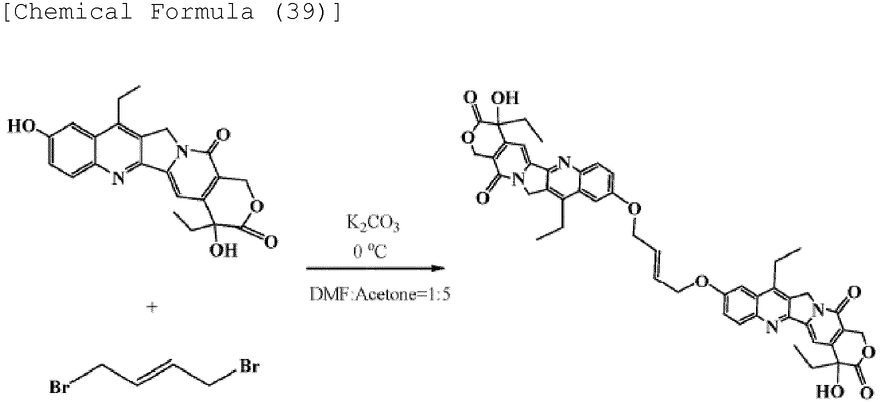

SN-38 (20 mg, 0.051 mmol) was placed in two-necked eggplant flask, and after nitrogen substitution, 0.3 ml of dehydrated DMF was added and stirred for 10 minutes to completely dissolve SN-38. 1,4-Dibromo-2-butene (5.4 mg, 0.025 mmol) was added thereto and stirred to dissolve, then dehydrated acetone 1.5 ml and potassium carbonate (18 mg, 0.127 mmol) were added, and stirred for two night.

The stirred solution was transferred to separatory funnel, ethyl acetate was added, and washed several times with aqueous ammonium chloride solution. The solution was separated into aqueous phase and organic phase, each placed in Erlenmeyer flask, and the aqueous phase was confirmed with thin-layer chromatography (TLC) and the organic phase was filtered through absorbent cotton after dried over magnesium sulfate. Then minimum amount of silica which was adsorbed to the filtrate was added, and the solvent was completely distilled away under reduced pressure. The residue was purified by silica gel column chromatography with chloroform/methanol = 10/1 as developing solvent. The color of the purified crystals was pale yellow, and the yield of dimer of SN-38 and 1,4-dibromo-2-butene (Compound 18-(d)) was 23%.
¹HNMR (400MHz, DMSO,δ) : 0.85 (t, J = 9.8Hz, 6H), 1.16 (t, J = 7.3 Hz, 6H), 2.02(q, J = 13Hz, 4H), 2.69 (q, J = 11.7Hz, 4H), 4.01 (s, 4H), 4.58 (d, J = 8.2Hz, 4H), 5.30 (s, 4H), 5.41 (s, 2H), 6.03 (s, 2H), 7.26(s, 2H), 7.52(s, 4H), 7.94 (s, 2H).

### Example 4 (comparative)

### Synthesis of betamethasone valerate dimer (Compound 19-(b)) using esterification

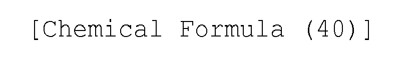

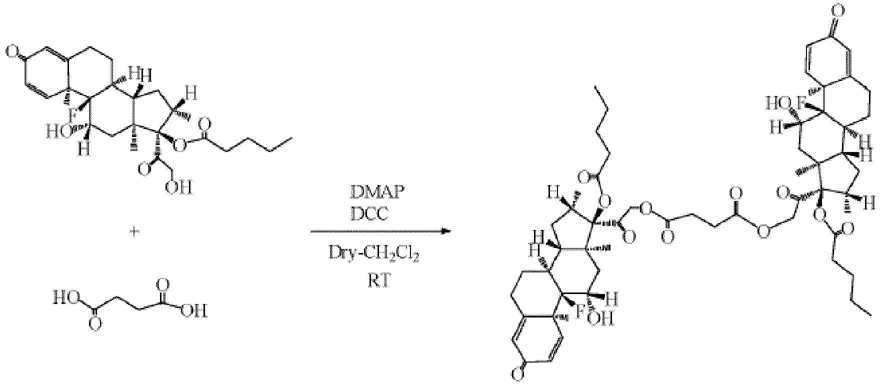

Succinic acid (36 mg, 0.286 mmol), 4-dimethylaminopyridine (DMAP: 174 mg, 1.431 mmol) and N,N-dicyclohexylcarbodiimide (DCC: 132 mg, 0.629 mmol) were placed in two-necked eggplant flask, substituted with nitrogen gas, dehydrated dichloromethane (0.1M, 6 ml) were added, and stirred for 30 minutes. Then, betamethasone valerate (300 mg, 0.629 mmol) was added thereto, and stirred overnight. The stirred solution was filtered through Celite to remove the precipitates of urea, and the solvent of the filtrate was completely distilled away under reduced pressure. Chloroform was added thereto to dissolve the products, minimum amount of silica which was adsorbed to the solution was added, and the solvent was distilled away under reduced pressure. Further the silica was dried to be a dry powder by completely removing the remaining solvent using vacuum line, and purified by silica gel column chromatography using ethyl acetate as developing solvent. The color of the purified crystals was white, and the yield of dimer (Compound 19-(b)) was 53%.
¹HNMR (400MHz, DMSO, δ) : 0. 91 (t, J = 7.3Hz, 6H), 0. 93 (d, J = 9.8 Hz, 6H), 1.26(ddd, J = 13.6Hz, 4H), 1.33(d, J = 7.8Hz, 6H), 1.35(q, J = 7.8Hz, 6H), 1.56(d, J = 8.8Hz, 6H), 1.58(s, 2 H), 1.60(q, J = 5.8Hz, 4H), 1.62(d, J = 5.8Hz, 2H), 1.64(q, J = 9.8Hz, 4H), 1.99(q, J = 10.2Hz, 2H), 2.12(t, J = 8.8Hz, 2H), 2.34(t, J = 7.8Hz, 4H), 2.41(t, 8.8Hz, 4H), 2.49(q, J = 6.8Hz, 2H), 2.63 (s, 2H), 2.78(t, 5.9Hz, 4H), 4.40(t, 7.8Hz, 2H), 4.84 (s, 4H), 6. 14 (s, 2H), 6. 34 (s, 2H), 6.36 (s,2H)
MS (ESI) : m/z =1035 (M+H⁺), 1057 (M+H⁺+Na⁺)

### Example 5

### How to make nanoparticles

### <ester bond of SN-38>

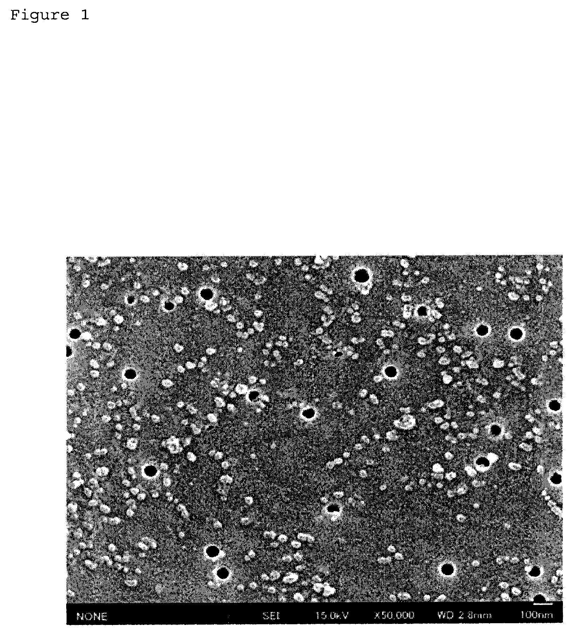

Dimer of SN-38 and succinic acid was weighed out to be 5 mM, and dissolved in DMSO 150 *µ*l. Using water as poor solvent, 100 *µ*l of the DMSO solution was injected into water 10 ml with syringe at room temperature, thus aqueous dispersion solution of dimer nanoparticles was prepared. Final concentration of the aqueous dispersion solution was 0.05 mM. The particle size was 50 nm (Figure 1).

### Example 6

### How to make nanoparticles

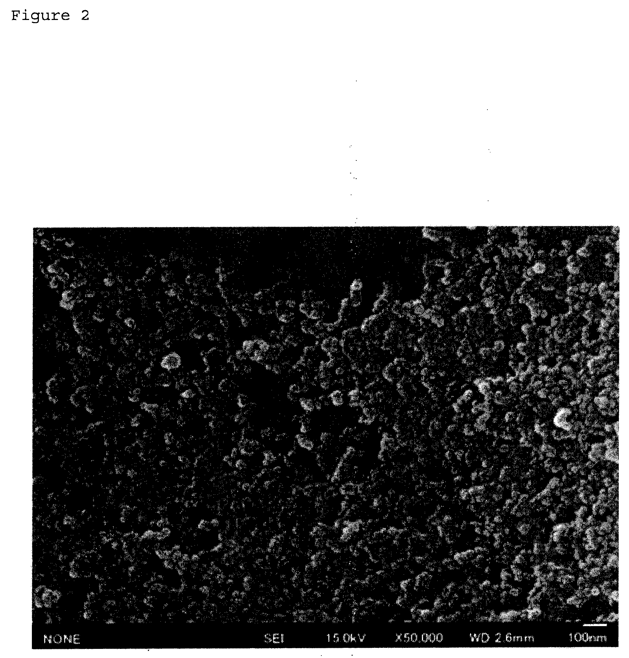

Dimer of SN-38 and tetramethylene diisocyanate was weighed out to be 5 mM, and dissolved in 150 *µ*l of DMSO. Using water as poor solvent, 100 *µ*l of the DMSO solution was injected into water 10 ml with syringe at room temperature, thus aqueous dispersion solution of dimer nanoparticles was prepared. Final concentration of the aqueous dispersion solution was 0.05 mM. The particle size was 50 nm (Figure 2).

### Example 7 (comparative)

### How to make nanoparticles

Dimer of betamethasone valerate and succinic acid was weighed out to be 5 mM, and dissolved in 150 *µ*l of DMSO. Using water as poor solvent, 100 *µ*l of the DMSO solution was injected into water 10 ml with syringe at room temperature, thus aqueous dispersion solution of dimer nanoparticles was prepared. Final concentration of the aqueous dispersion solution was 0.05 mM. The particle size was 150 nm (Figure 2).

### Example 8

### In vitro anticancer activity of various SN-38 dimer nanoparticles

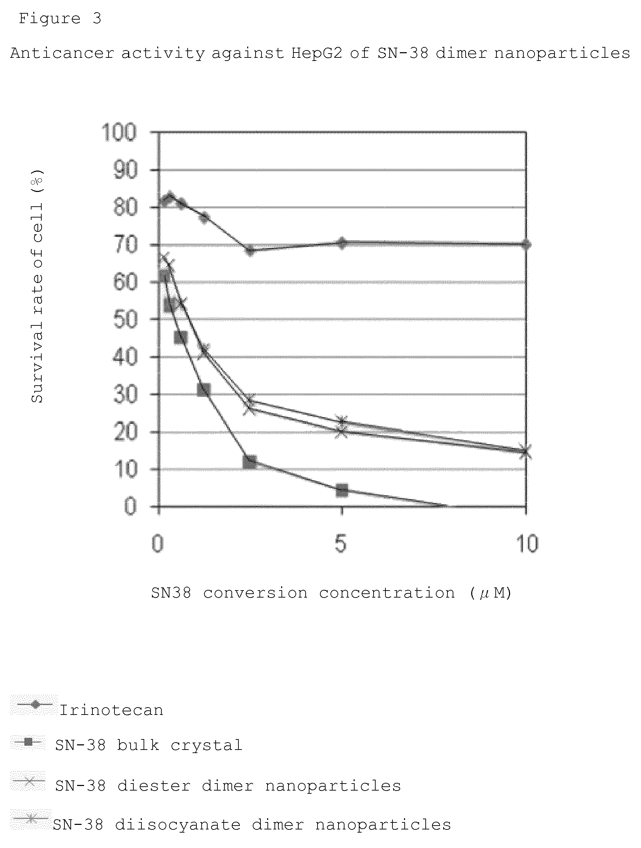

Human liver cancer cell lines HepG2 was seeded on 96-well plate in 2x10⁴ cells/well. Next day, irinotecan, SN-38 bulk crystal, SN-38 diester dimer nanoparticle, and SN-38 diisocyanate dimer nanoparticle were added to the HepG2 cell culture in an amount to be 0.15625, 0.3125, 0.625, 1.25, 2.5, 5 or 10 µM in terms of SN-38 monomer. After cultivating for 48 hours, the cell viability was measured by a colorimetric method using Cell Counting Kit-8 (manufactured by DOJINDO) and microplate reader (manufactured by BIO-RAD). As the result, under the present condition, irinotecan showed almost no anticancer activity, whereas various SN-38 samples showed remarkable anticancer activity. In addition, it became obvious that when SN-38 was dimerized via ester bond or urethane bond, or further nanoparticulated, the anticancer activity thereof can be retained (Figure 3).

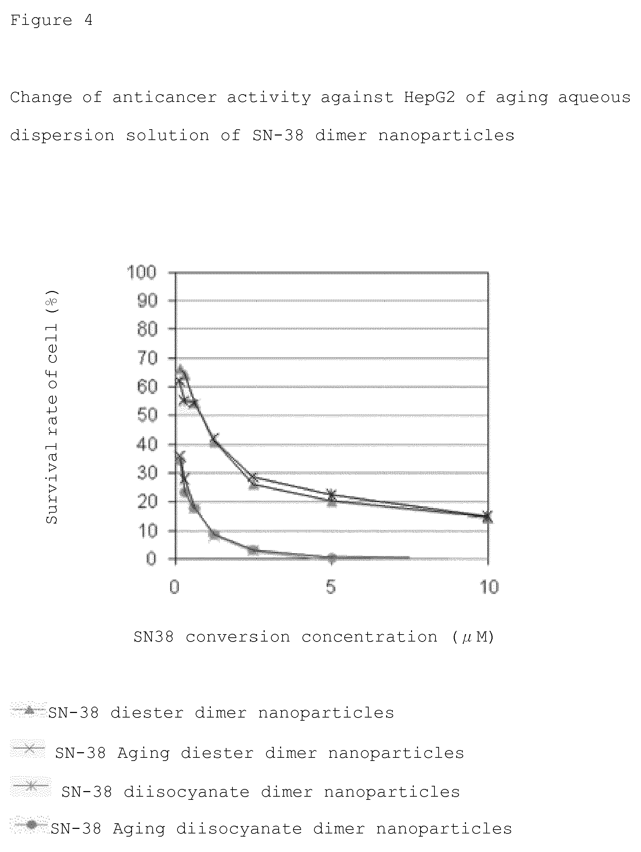

Further, two kinds of aqueous dispersion solution of SN-38 dimer nanoparticles were left at room temperature for about one week, thus prepared aging sample. It was revealed from the study about aging effect as an indicator of anticancer activity against HepG2 cell that in both of the two kinds of SN-38 dimer, the anticancer activity is increased by aging (Figure 4).

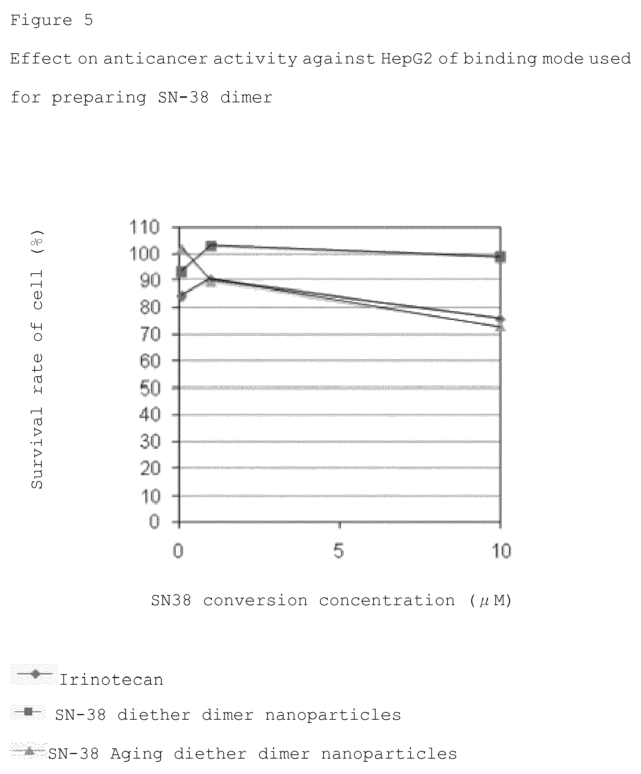

In Figure 3 and Figure 4, there was no difference in the anticancer activity against HepG2 between the binding modes included in the SN-38 dimer nanoparticles, namely, ester bond and urethane bond. That is, both bonds are expected to be decomposed at about the same speed in the solution, and SN-38 monomers are formed. To confirm this, SN-38 is dimerized with low degradable ether bond, further subjected to aging, then studied the anticancer activity against HepG2. The treatment concentration at this time was made 0.1, 1, 10 µM in terms of SN-38. As the result, the anticancer activity of the SN-38 diether dimer nanoparticles almost disappeared, and also the aging effect was little (Figure 5). The above results suggested that the anticancer activity of the SN-38 dimer nanoparticles depends on the decomposition rate of the dimer. On the contrary, it suggests that the anticancer activity of the SN-38 dimer nanoparticle can be controlled by the binding mode used for dimerization, which is considered to be useful for adjusting the medicinal effect expression profile after in vivo administration.

## Claims

1. A solid pharmaceutical nano particle of a compound selected from the group consisting of 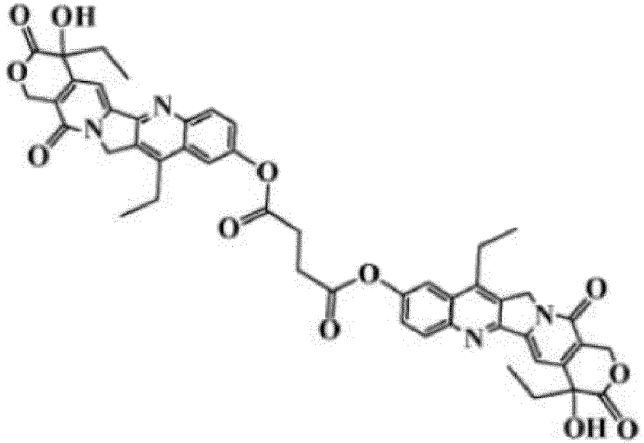 and 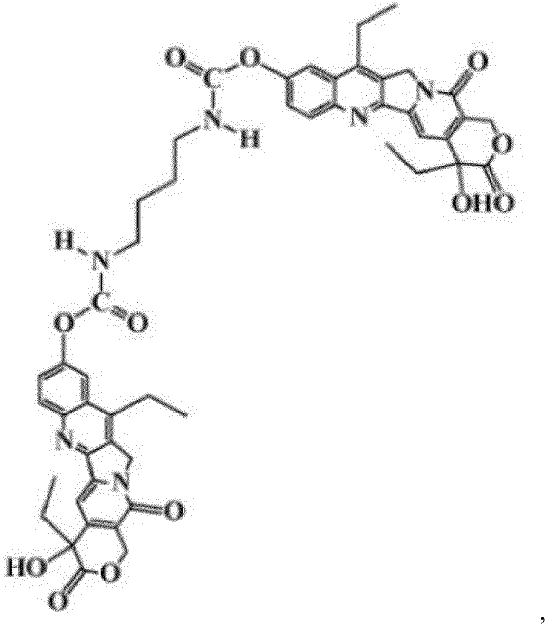 which is **characterised by** being obtained by pouring a solution of the compound dissolved in a water-miscible organic solvent into water.

2. The solid pharmaceutical nano particle according to claim 1, wherein the compound is

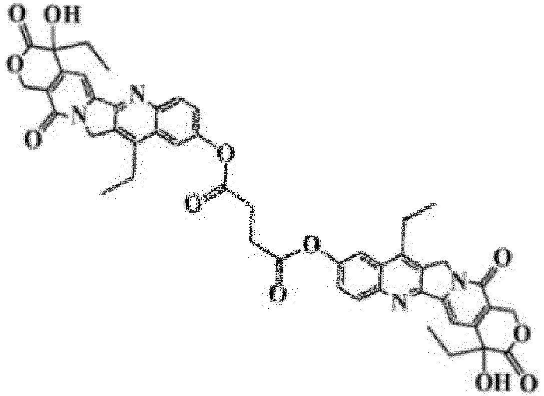

3. An anti-cancer agent comprising the solid pharmaceutical nano particle according to claim 1, as an active ingredient.

4. A manufacturing method of the solid pharmaceutical nano particle according to claim 1, which is **characterised by** pouring a solution of the compound selected from the group consisting of 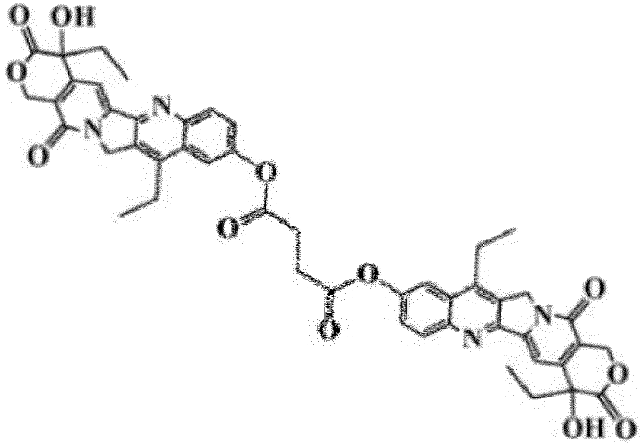 and 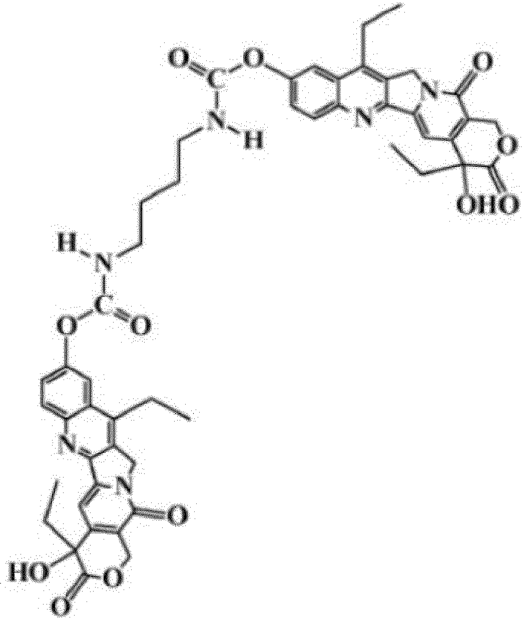 dissolved in a water-miscible organic solvent into water.

## Patentansprüche

1. Ein fester pharmazeutischer Nanopartikel eines Stoffesausgewählt aus der Gruppe bestehend aus 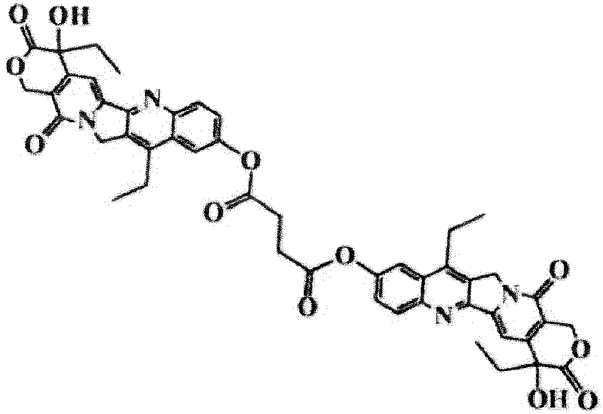 und 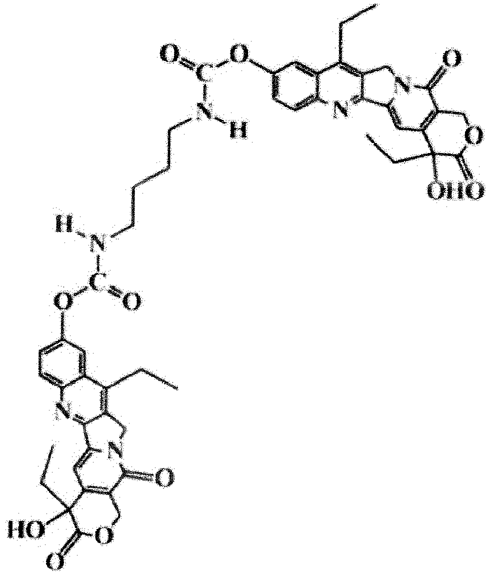 charakterisiert dadurch, dass er durch das Einschütten einer Lösung des Stoffes, welcher in einem wassermischbaren organischen Lösungsmittelgelöst ist, in Wasser erhalten wird.

2. Der fester pharmazeutische Nanopartikel gemäß Anspruch 1, wobei der Stoff 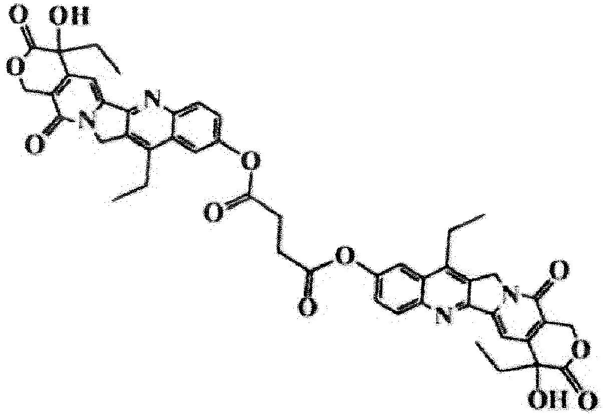 ist.

3. Ein Anti-Krebs Mittel, welches den festen pharmazeutischen Nanopartikel gemäß Anspruch 1 alls aktiven Wirkstoff enthält.

4. Verfahren zur Herstellung des festen pharmazeutischen Nanopartikels gemäß Anspruch 1, welches **dadurch gekennzeichnet ist, dass** eine Lösungeines wassermischbaren organischen Lösungsmittels und dem gelösten Stoff ausgewählt aus der Gruppe bestehend aus 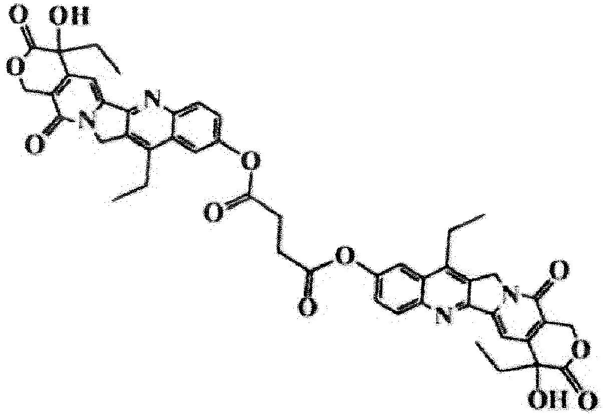 und 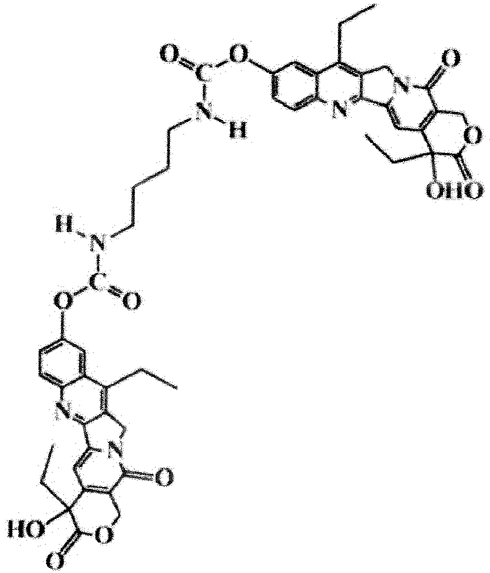 in Wassereingeschüttet wird.

## Revendications

1. Nanoparticule solide pharmaceutique d'un composé choisi dans le groupe constitué de 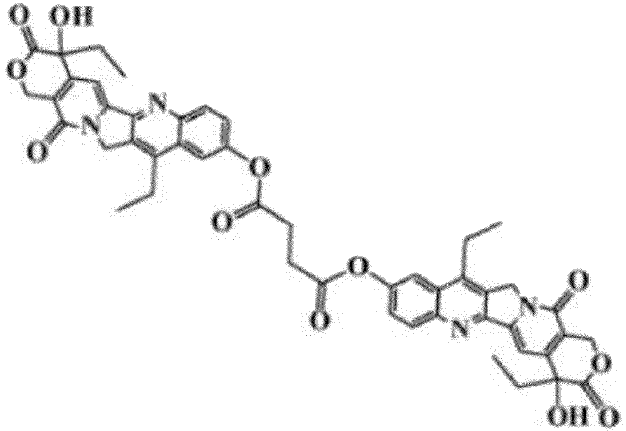 et 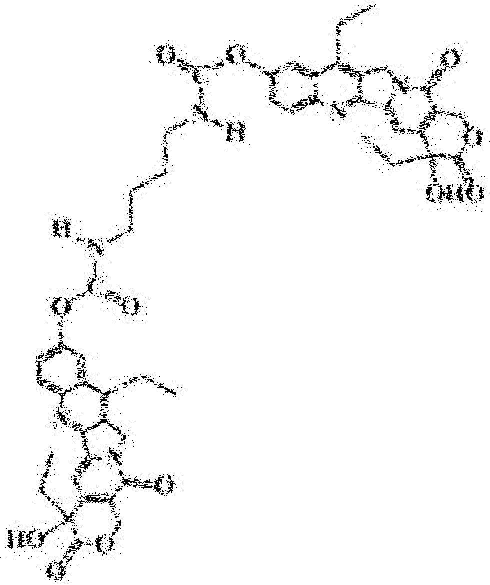 qui est **caractérisée en ce qu'**elle est obtenue en versant une solution du composé dissous dans un solvant organique miscible à l'eau dans de l'eau.

2. Nanoparticule solide pharmaceutique selon la revendication 1, dans laquelle le composé est

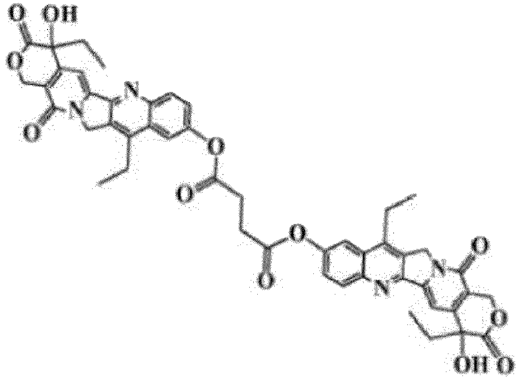

3. Agent anticancéreux comprenant la nanoparticule solide pharmaceutique selon la revendication 1, en tant que principe actif.

4. Méthode de fabrication de la nanoparticule solide pharmaceutique selon la revendication 1, qui est **caractérisée par** le versement d'une solution du composé choisi dans le groupe constitué de 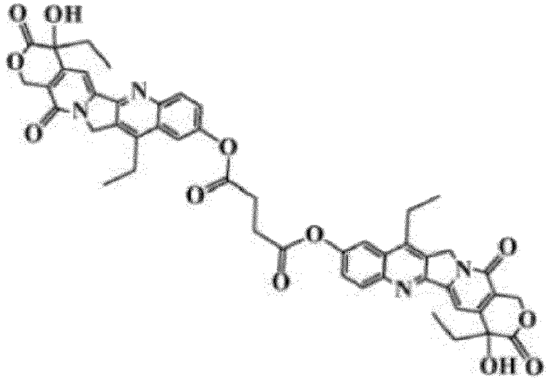 et 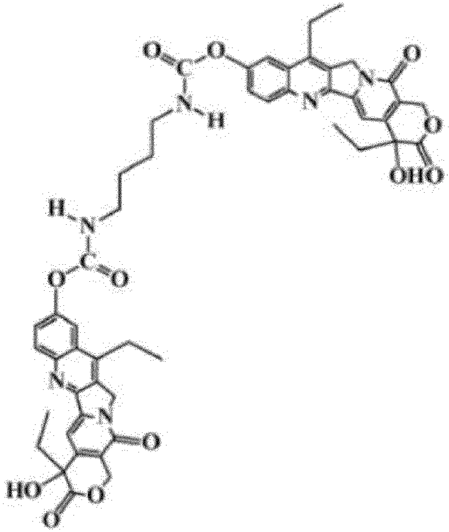 dissous dans un solvant organique miscible à l'eau dans de l'eau.
